(19)

**Europäisches Patentamt**
**European Patent Office**
**Office européen des brevets**

(11) **EP 1 932 592 A1**

(12) **EUROPEAN PATENT APPLICATION**

(43) Date of publication:
**18.06.2008 Bulletin 2008/25**

(51) Int Cl.:
*B01J 29/85* (2006.01)          *B01J 29/90* (2006.01)
*C01B 37/08* (2006.01)          *C07C 41/09* (2006.01)
*C07C 43/06* (2006.01)

(21) Application number: **06025797.9**

(22) Date of filing: **13.12.2006**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK RS**

(71) Applicant: **Casale Chemicals S.A.**
**6900 Lugano-Besso (CH)**

(72) Inventor: **Ferrini, Cristina**
**6932 Braganzona (CH)**

(74) Representative: **Zardi, Marco**
**M. Zardi & Co. S.A.**
**Via Pioda 6**
**6900 Lugano (CH)**

(54) **Process and catalyst for the production of dimethylether**

(57)    The present invention relates to a process for the production of DME characterized by the step of dehydrating methanol in vapor phase in the presence of a catalyst comprising, as a active component, a silicoaluminophosphate (SAPO) molecular sieve having a minimum medium acidity of 0.5-1 mmol for gram of dry sieve at a temperature in the range from 160°C to 280°C. The invention also relates to said catalyst as well as processes for its preparation and regeneration.

Figure 1

EP 1 932 592 A1

**Description**

Field of application

In its most general aspect, the present invention relates to a process for the production of dimethylether (DME) from methanol.

In particular, the present invention relates to a process for the production of DME from methanol using a catalyst based on silicoaluminophosphate (SAPO) molecular sieves.

The present invention further relates to a catalyst based on silicoaluminophosphate molecular sieve suitable for use in the production of DME from methanol as well as processes for the preparation and regeneration of said catalyst.

Prior Art

DME is a chemical known for many years and it is used for many applications.

In particular, fuel grade DME (88.9 %) is used as multipurpose fuel. DME has the potential to become widely used as a domestic fuel for heating and cooking (bottled), as a power generation fuel, as a clean diesel alternative, as a chemical feedstock and in fuel cells cars.

As a chemical feedstock, DME is used for the manufacture of fuel additives and as a preferred feedstock in olefins production.

Highly pure DME (99.9%) is suitable for the production of aerosols, that are used as a propellant for the preparation of deodorants, hair sprays, pharmaceutical products, insecticides and paint cans, as well as for other applications as refrigerant in cooling devices and as blowing agents in plastic foams.

The characteristics of DME as a fuel are more interesting than those of methanol because it is a no toxic gas and, owing to the loss of water, it has a higher heat value ($LHV_{DME}$: 6900 kcal/kg, $LHV_{CH3OH}$: 4800 kcal/kg) .

DME is physically similar to liquefied petroleum gas (LPG) thus it can take advantage of the vast experience in LPG handling. DME can be transported using conventional LPG tankers and stored at a receiving location using the same conventional unloading and storage equipment as LPG-type with only small alterations. Table 1 shows the properties of DME compared with those of propane and butane, that are main components of LPG.

Table 1 Properties of DME and of Propane and Butane

| Property | DME | Propane | Butane |
|---|---|---|---|
| Boiling point °C | -25 | -42 | -0.5 |
| Vapour pressure @ 20 °C | 5 | 8 | 2 |
| Liquid density @ 20 °C kg/m$^3$ | 670 | 500 | 610 |
| Specific density gas | 1.6 | 1.5 | 2.0 |
| LHV kcal/kg | 6900 | 11'100 | 11'000 |
| Ignition temperature at 1 bar, °C | 235-350 | 470 | 365 |
| Flammability limit in air, vol % | 3.4 - 17 | 2.1- 9.4 | 1.9 - 8.4 |

It will also be possible to introduce, under appropriate conditions DME into the methane pipeline network.

Up to about ten years ago, chlorofluorocarbons (CFCs) were used for all the aerosol and refrigerant applications because they remained unreactive and were therefore safe.

The danger of a global earth warming due to the greenhouse effect and the destruction of the ozone layer, in particular the Antarctic ozone hole, have imposed the necessity to initiate in depth studies to identify the causes of these phenomena. The results are briefly summarized in Table 2.

Table 2: Main pollutants responsible for the greenhouse effect and of the depletion of the ozone layer.

| Product | $CO_2$ | $CH_4$ | CFC |
|---|---|---|---|
| Potential of radiative activity compared to $CO_2$ | 1 | 32 | 1500 |
| Half time (years) | 50-200 | 10 | 60-400 |
| Effect on global Earth warming in 20 years | 1 | 63 | 7000 |

Table 2 shows that the CFCs are indeed linked to the ozone depletion and to the greenhouse effect. For this later aspect the more dangerous chemicals are carbon dioxide and methane.

According to international legislation and agreements it has been recommended to decrease the emissions of $CO_2$ and $CH_4$ and to ban the production and use of CFCs from the year 2000. In the industrialized countries the CFCs are banned from the 80.

Substitutes of the CFCs were developed by the industry since the volatile organic substances are essential for the manufacture of aerosol, refrigerants and plastic foams. Up to now the best substitutes of the CFCs are the low molecular weight paraffins (propane, butane and pentane) and DME.

However, if compared to the paraffins DME has the following advantages:

- the heating value is two times lower

- its lower explosive limit in air is higher

- it is soluble in organic solvents and water

- it can be synthesized at a high degree of purity, higher than 99.9 %

- it is a colourless and odourless gas

- its toxicity is very low

- it is environmentally benign as it is not harmful to the ozone layer and do not influence the greenhouse effect

Because of those advantages the DME is considered the best substitute of the CFCs in the aerosols.

With regard to its production, DME may be easily manufactured by the usual ether synthesis paths. For industrial production, DME can be most economically produced by acid catalysed dehydration of methanol, both in liquid and vapour phase.

In liquid phase, catalysts are strong mineral acids as sulphuric and phosphoric acid. In gas phase, solid acid catalysts are used, as alumina, silica-alumina, activated alumina, diatomite treated with phosphoric acid, clays, sulfates, acid resins and molecular sieves (natural or synthetic). The necessary temperatures are higher than 270 °C. (U. S. Patent No. 4,590,320).

The liquid phase technologies need high pressures and the quantitative separation of the catalysts is difficult, therefore there is always the risk of the hydrolysis of the ether to methanol, which is toxic by skin absorption and may cause serious eye damage. Furthermore, the presence of methanol in aerosols used for cosmetic, body care sprays or pharmaceutical products has a maximum limit of 10 ppm.

The vapour phase process may be conducted, with commercially acceptable conversions, at temperatures around 300 °C. For industrial use, the processes for the DME synthesis are based on aluminium oxide or aluminium silicate catalysts at 300 °C.

However, due to thermodynamic limitations, at this temperature the amount of unconverted methanol is higher than 15%.

DME is also a primary product in methanol conversion to hydrocarbons over molecular sieve catalysts having acidic sites (see for instance G.H. Hutchings, R. Hunter, Catal. Today, Vol. 6, 279 (1990)). However, these molecular sieves have strong acidic sites ( Bronsted acidic sites) that can catalyse the formation of non-volatile hydrocarbons at temperatures as low as 250 °C, this being the limit for which methanol starts being converted to hydrocarbons on acidic dehydration catalysts (see H. Schulz, w. Boehrigeren and S. Zhao, Proceedings of the 9th Int. Molecular sieve Conf., Vol. 2, 567 (1993)).

As a result, with the known molecular sieves acidic dehydration catalysts, hydrocarbons are present in the reaction's products that are environmentally unacceptable or very toxic as for instance methane or benzene. Moreover, in order to obtain DME of very high purity, higher than 99.99 %, and a methanol and hydrocarbons level in the order of several tenths of parts per million (ppm), complex and expensive separation and purification systems are needed, that may comprise up to four distillation columns and related equipment as pumps, reboilers, condensers and reflux pumps. In some cases chemical treatment phases are also needed.

The presence of by-products, the separation and the recycle of the unconverted methanol to the synthesis reduce the production capacity of the plant and increase the utilities specific consumption, thus increasing the production costs of DME.

Therefore, a drawback of conventional acidic dehydration catalysts is that, due to their strong acidity, they catalyse at the same time side reactions, resulting in an undesired high content of by-products.

Another drawback of the known acidic dehydration catalysts is their short life because of rapid deactivation by deposition of coke consisting of polymerization products and/or carbon (unsaturated hydrocarbons). The deactivation of the catalyst is an accelerating process which rapidly involves the catalyst regeneration or replacement.

Furthermore, with the known acidic dehydration catalysts, it is only possible to closely approach the equilibrium at a temperature around 300 °C, thus hydrocarbons formation cannot be avoided. Thermodynamically the equilibrium conversion is higher and the selectivity tends to be improved at lower temperatures.

Numerous attempts have been made at bringing about improved properties to the above acidic dehydration catalysts by varying the pore structure and/or by special treatments causing atoms of other elements to form part of the structure or by washing with bases or by ions exchange. However, until now these efforts have been without much success. Some cations that exchange Bronsted sites, were, in fact found to retard the rate of deactivation and to stabilize the molecular sieve but also decreased its activity (see for instance: Applied Catalysis A, 276 (2004) 251-255, Fuel Processing technology, 83 (2003) 203-218, Microporous and Mesoporous Materials, 29 (1999) 3-48, US6936566, US6514899 and Catalysis communications 6 (2005) 147-152).

Summary of the invention

The technical problem underlying the present invention is that of providing a process for production of DME from methanol using a dehydration acidic catalyst which overcomes the mentioned drawbacks with reference to the prior art.

This problem is solved, according to the present invention, by a process for the production of DME characterized by the step of dehydrating methanol in vapor phase at a temperature in the range from 160°C to 280°C in the presence of a catalyst comprising, as a active component, a silicoaluminophosphate (SAPO) molecular sieve having a minimum medium acidity of 0.5-1 mmol for gram of dry sieve.

In the process according to the invention, the methanol feed may be a vapor flow consisting essentially of methanol, as for example commercial methanol having a minimum methanol content of 99.8% or a vapor flow comprising methanol as a main component, for example a mixture of methanol and steam. In the case of a mixture of methanol and steam, the steam content should not exceed preferably 15% by weight.

Preferably, in the process according to the invention, the above dehydration step is carried out at temperatures in the range from 160°C to 250°C, while preferably 180°C to 220°C.

The process of this invention achieves considerable advantages over the prior art since no aromatic hydrocarbons are formed in the methanol conversion to DME and the amount of methane formed is very low, never exceeding 5 % of the total quantity of the hydrocarbons formed.

In addition, using a catalyst comprising SAPO molecular sieves having an acidity as specified above, it is possible to carry out the process at optimum conditions, in particular at lower temperatures than those of the prior art processes. As a result, the selectivity for methanol conversion to DME is very high, in particular it is possible with the process according to the invention to obtain a selectivity for DME close to 100 % and conversions of methanol that approach the thermodynamic equilibrium by 90 % and more.

In particular, to maintain the catalyst high selectivity the temperature should not exceed 280 °C, while at 160 °C the catalyst is already active.

Furthermore, in the process according to the invention, the quantity of methanol to be recycled (unconverted methanol) is lower than 15%, this increases the plant productivity by more than 50% and reduces by around 25% the utilities specific consumption. The performances of the catalyst are not essentially modified if the methanol contains up to 15% of water.

The present invention also relates to a catalyst for the conversion of methanol to DME comprising, as an active component, a silicoaluminophosphate (SAPO) molecular sieve having a minimum medium acidity of 0.5-1 mmol for gram of dry sieve.

Preferably, the SAPO molecular sieve is a SAPO-34 molecular sieve.

According to the invention, the molecular sieve of the above catalyst is obtained by hydrothermal treatment of a suspension obtained, in turn, by mixing of a Al source, such as hydrated alumina or aluminium nitrate with a silica sol and phosphoric acid and with a templating agent as aliphatic amines or quaternary ammonium salts with appropriate molar ratio between the above reagents.

The term "templates" typically refer to structure directing agents that are used to form channels or tunnel-like structures (also called microporous structure) within the molecular sieve composition. For the SAPO materials to be used as catalyst compositions, however, the template must be removed to open up the channels or tunnel-like structures.

Preferably, appropriate molar ratios of the above reagents in the final mixture expressed in terms of P, Al and Si oxides and using TEAOH (tetraethyl ammonium hydroxide) as a templating agent are as follows $P_2O_5 : Al_2O_3 : SiO_2 :$

TEAOH : $H_2O$ = 1 : (1.0-1.4): (0.2-0.4) : (1.0-1.15) : (110-130)

Preferably, all said reagents have a very low Na content (< 0.01 %).

According to an aspect of the present invention, the templating agent TEAOH is first mixed with phosphoric acid aqueous solution to obtain tetraethylammonium phosphate (TEAP) in aqueous solution and then hydrated alumina and silica sol are added to the latter solution.

To obtain the catalyst according to the invention, the molecular sieve obtained as above typically in form of a suspension, is recovered by said suspension in a conventional way (for example by filtration or centrifugation), dried and mixed or inserted in a matrix material that acts as a diluent but also as a continuous binder phase between the molecular sieve crystallites to form a paste. This paste is then treated to form solid particles by extrusion, compression techniques or atomisation to form extrudates, pellets or microspheres and these solid particles are subjected to a calcination procedure.

Preferably, the mixture contains 70-98% by weight of matrix material and 2-30% by weight of molecular sieve, most preferably, 90-98% by weight of matrix material and 2-10% by weight of molecular sieve.

Suitable matrix material that does not affect the catalytic properties of the molecular sieve are activated alumina, silica, aluminium silicate, silica-alumina and natural caoline. Preferably, said matrix materials have a high surface area and good binding properties given from reactive surface groups.

Preferred matrix materials consist of activated aluminas having at least 50% by weight of pseudoboehmite and more than 100 $m^2$/g surface area.

According to an aspect of the present invention, the calcination of the mixture of molecular sieve and matrix material is carried out at temperatures up to 600°C. Preferably, said calcination is started at ambient temperature and the temperature is gradually increased in 4-10 hours up to 600°C and kept at 600°C for further 4-8 hours.

According to a preferred aspect of the present invention, said calcination is carried out in presence of an inert gas as argon or nitrogen up to 500°C.

In this way, it has been surprisingly found that the calcination's procedure is shorter and can be achieved at lower temperatures while at the same time the inert gas acts a sort of stripping agent that allows the template to be removed in a substantially complete way. Furthermore, the catalyst so obtained shows a higher resistance and lifetime during its use in the conversion of methanol to DME.

According to another aspect of the present invention, the catalyst is in the form of pellet or extrudates having 1-6 mm diameter and 1-5 mm length.

According to a further aspect of the present invention, the catalyst is in the form of microspheres having medium diameter form 0.05 to 0.08 mm.

According to the invention, the process for converting methanol to DME may be performed in a reactor having a fixed bed or a fluidized bed of said catalyst. The reactor may be isothermal with cooling surfaces in direct contact with the shaped catalyst or adiabatic with a plurality of catalytic beds and intercooling between the beds.

Preferably, the operating pressure in the reactor is in the range from atmospheric pressure to 20 bar, preferably 5 to 15 bar and the methanol flow has a space velocity, expressed as liters of liquid methanol supplied for liters of catalyst volume, in the range of 1 to 5, preferably 1.5 to 3.5.

For economic reasons, the operating pressure is chosen so that the DME can be condensed, at the fractionation column exit, by the use of industrial recycle water at 28 °C.

In an embodiment of the present invention, the reaction products are fed as vapour streams to the fractionation column at the reaction pressure chosen so that the above conditions are met. Thereby utilities and energy savings are obtained since the compression of the vapours to the separation and the evaporation of the raw materials fed to the reaction products separation section are not needed any more.

Because the performance of the catalyst is not affected by the presence of water in the raw materials, the recovery column for the residual methanol in the reaction products can operate under conditions that are convenient from an economic point of view, in fact methanol may be recycled with a water content as high as 15 %.

The reaction products separation unit has two distillation columns connected in series, that have although very flexible operating parameters, so that the useful products, respectively, DME and recycle methanol, can be withdrawn very pure as side streams from the two columns, and the by-products and water, are respectively taken from the top and from the bottom of the columns.

The separation and purification of DME downstream of the reaction section may follow two separate, while interconnected processes, for the Fuel and for, respectively, the Aerosol Grade product.

The present invention further relates to a process for regenerating the catalytic activity of a catalyst as described above after each cycle of its use in a process according to the invention for converting methanol to DME, the regenerating process comprising the step of passing across the catalyst at least one flow of a regenerating agent chosen among nitrogen, argon, hydrogen, air, steam and mixtures thereof at a temperature of maximum 500°C for a period from 6 to 12 hours.

Further characteristics and advantages of the process and of the catalyst according to the invention for the production of DME from methanol will be more evident from the examples provided here below, given as indicative and not limiting purpose.

Brief description of the drawings

Figure 1 shows a) the thermogravimetric analysis (TGA) of a SAPO-34 molecular sieve of a catalyst according to the invention compared with b) the thermogravimetric analysis of a SAPO-34 that is not suitable to be used as a catalyst for DME synthesis.

Figure 2 shows the influence of the molecular sieve content in the catalyst of the invention on the DME production yield.

Figure 3 shows the influence of the temperature on the DME yield and methanol conversion using a catalyst according to the invention.

Figure 4 shows a flow sheet of a DME synthesis plant implementing the process according to the invention and gives a material balance around the main equipment.

Figure 5 shows the thermogravimetric analysis of the as-synthesized SAPO-34 molecular sieve according to the invention in air.

Figure 6 shows the thermogravimetric analysis of the as-synthesized SAPO-34 molecular sieve according to the invention in argon.

Figure 7 shows a superposition of the thermogravimetric analysis of Figures 5 and 6 of the as-synthesized SAPO-34 molecular sieve in air and in argon respectively.

Figure 8 shows the results of a lifetime experiment with a catalyst according to the invention over six days, giving the methanol conversion.

Figure 9 shows the thermogravimetric analysis of the SAPO-34 molecular sieve used in lifetime experiments in air, dry argon and saturated argon.

Example 1

An initial reaction mixture having the molar composition 1.63 $Al_2O_3$ : 0.33 $SiO_2$ : 1.0 $P_2O_5$ : 1.65 TEAOH : 129 $H_2O$ (TEAOH is tetraethyl ammonium hydroxide) was prepared. This operation is made at 20-30 °C and normal pressure, by mixing in a 5000 liter autoclave, under continuous stirring, 210 kg hydrated alumina 63% $Al_2O_3$, with 52 kg silica sol (30 % wt, ammonia stabilized) and addition of 1143 kg of tetraethylammonium phosphate in aqueous solution obtained from 183 kg orthophosphoric acid 84 % and 960 kg tetraethylammonium hydroxide (20%). To obtain the prescribed water concentration in final reaction mixture a subsequently water quantity is added. Total demineralised water used in this phase is 890 kg. The duration of the gel formation and maturation period is about 14 hours.
The initial gel suspension has to have a pH value of 6.2-6.5.
After this gel formation period the synthesis by hydrothermal treatment at 190-195 °C is carried out by heating the autoclave at crystallization temperature in about 2 hours and maintaining this temperature 12 hours. During the crystallization reaction phosphorous atoms enter in the molecular sieve framework and release a corresponding free tetraethylammonium hydroxide. Consequently the pH of the reaction mixture increases to 8.0-8.5 where the crystallization process almost stops. To compensate this effect a stepwise pH correction is performed.
The first crystallization is followed by the autoclave cooling at room temperature, first pH correction with a suspension containing 10 kg silica sol., 30 kg orthophosphoric acid, 42 kg hydrated alumina and 325 kg demineralised water, autoclave reheating to 190-195 °C crystallization temperature in two hours and 12 hours second crystallization step. The second and third pH correction steps are made with 130 kg phosphoric acid previously diluted to 15%. The crystallization steps are conducted in the same manner as already described. So the complete crystallization phase duration is of about 72 hours.
The final slurry composition was as follows: 1.36 $Al_2O_3$ : 0.27 $SiO_2$ : 1.0 $P_2O_5$ : 1.14 TEAOH : 118 $H_2O$
SAPO-34 is obtained as an aqueous suspension that is separated by centrifugation in a 900 rpm centrifuge during 8-10 hours and discharged in a washing vessel. Two washings with 1600 kg demineralised water are made. Each washing is followed by separation in the same centrifuge. 650 kg of paste containing 315 kg water are obtained.

The material is dried in a steam heated dryer for 16 hours. TGA shows that the as synthesized SAPO-34 contains about 19% TEAOH trapped in its pores. To obtain the final catalyst composition the 335 kg of SAPO-34 are mixed with 1250 kg hydrated alumina and malaxate with 660 kg of nitric acid (10%). The resulting consistent paste is extruded in an extruding machine equipped with automatic cutting and collecting devices.

The extruded catalyst is dried for 6 hours at 100-110 °C in air.

The finite catalyst is obtained by calcining the extrudate in air up to 600°C for 14 hours. Approximately 1000 kg of finite catalyst are obtained.

The as synthesized molecular sieve is characterized by three analyses:

- Phase purity: X-ray diffraction spectra: crystallinity is greater than 90 %.

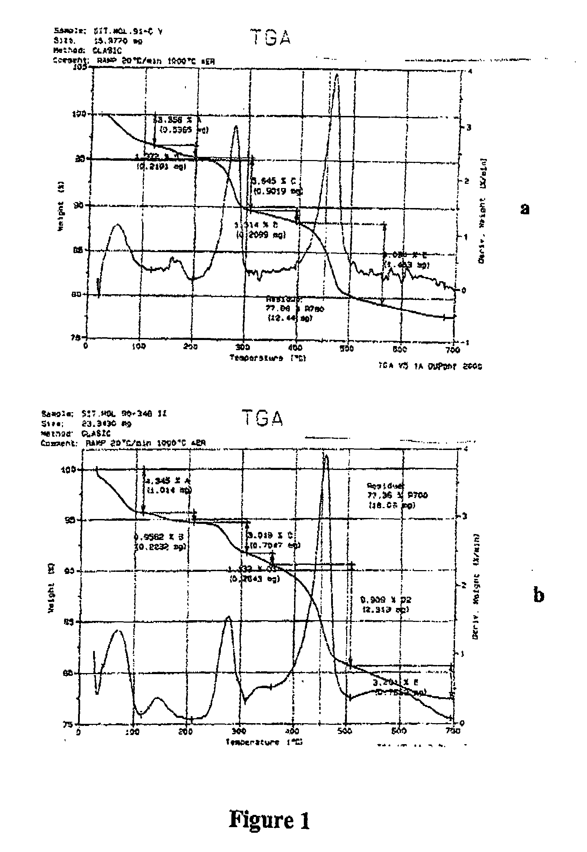

- Thermal analysis: weight lost at calcination is 21-23 %, and peaks are like in Figure 1a.

- SEM analysis: cubic crystals with crystallites no greater than 2 $\mu$m.

The final catalyst has the following characterization:

- Shape and dimension: cylindrical extrudates 2 x 4 mm

- Bulk density: 0.6-0.7

- Mechanical resistance (at cleavage): min 3 kgf/cm$^2$

Figure 1 also shows the medium acidic character of the as-synthesized SAPO-34 molecular sieve (see a)) and as a comparison the thermogravimetric analysis of a SAPO-34 that has a higher acidity and that is not suitable to be used as a catalyst for DME synthesis (see b)). Weaker acidic sites result in a maximum at lower temperature. The peak at about 250-300 °C indicates medium acidity whereas the peak at about 430-480 °C strong acidity.

The catalytic performances for the conversion of methanol to DME are as follows:

- activity: higher than 40 % methanol conversion (175 °C, 1 atm, 2h$^{-1}$)

- selectivity: higher than 99.8% dimethylether

Example 2

An initial reaction mixture having the molar composition 1.32 $Al_2O_3$ : 0.4 $SiO_2$ : 1.0 $P_2O_5$ : 1 TEAOH was obtained by suspension of 45 g of activated alumina of bayerite type in water (60%); the suspension is charged under stirring over a TEAOH solution containing 28.4 g TEAOH in 500 g water, and then 16 g of ammonium stabilized silica sol, having 30 % wt $SiO_2$ and 56 g of phosphoric acid at a concentration of 70 % wt were added.

After homogenization the reaction mixture was charged in a stainless steel autoclave and heated at 195 °C for 100 hours.

The reaction autoclave was cooled at room temperature, the molecular sieve product was filtered, washed with demineralised water dried for 4 hours at 105 °C, and finally calcined at 575 °C for 4 hours. The molecular sieve so obtained shows a medium acidity of 1 mmol /g molecular sieve and its pores are not accessible to benzene.

The calcined molecular sieve was mixed with hydrated alumina in different weight concentration of molecular sieve in alumina, i.e. 10 %, 20 % and 30 %, and was extruded to cylinders having 3 mm diameter and 3-6 mm height. The extrudate was dried for 4 hours at 105 °C and then calcined for 2 hours at 550 °C.

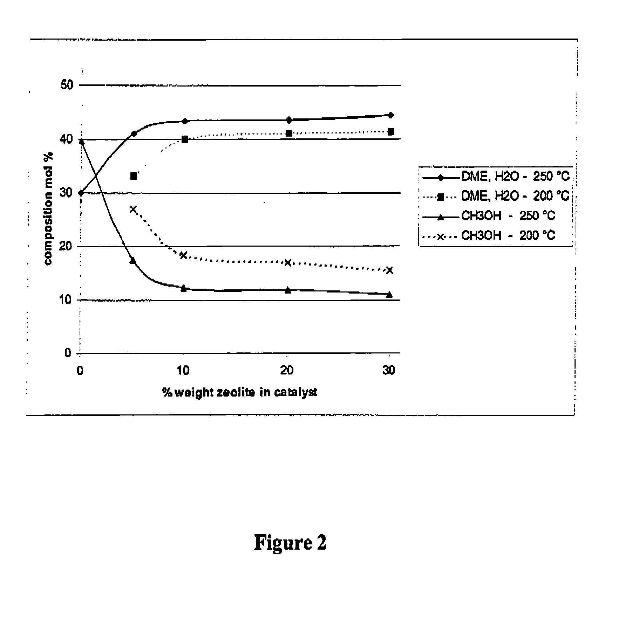

50 g of said catalyst were filled in a tubular type reactor. 100 g of 99.9 % methanol were fed to the reactor and have flown through the catalyst at 200 and 250 °C and 8 bar. The results obtained are given in Figure 2 and are compared to results obtained with an alumina catalyst without the molecular sieve phase. As it is shown by the diagram of Figure 2, thanks to the presence of the molecular sieve in the catalyst, methanol conversion is increased from 60 % (molar basis) to 80 % at 250 °C. At 200 °C the alumina shows a very low catalytic activity whereas the catalyst with the molecular sieve component shows conversion higher than 80 % (residual methanol 16-18 %) .

The only reaction products are DME and water according to the reaction:

$$2\ CH_3OH\ =\ CH_3OCH_3\ +\ H_2O\qquad <1>$$

No byproducts are formed under the operating conditions of example 2.

## Example 3

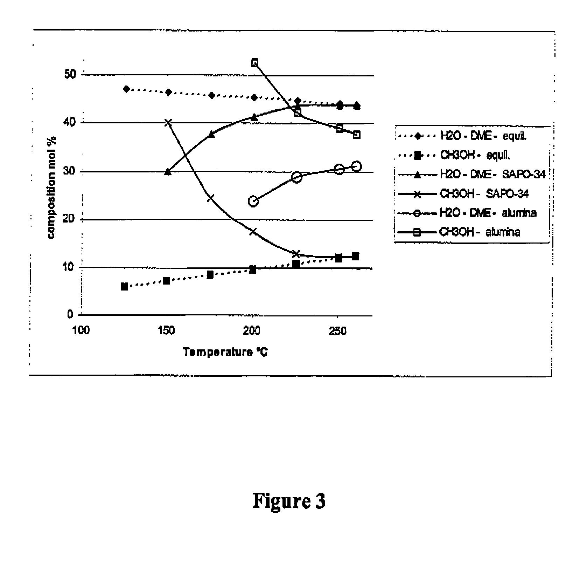

A catalyst according to the invention with a SAPO-34 molecular sieve content of 15 % was prepared according to the procedure of example 2. This catalyst has been tested in conditions as given in example 2, but varying the temperature over a wide range. The results obtained are shown in Figure 3 compared with the results obtained with activated alumina and the thermodynamic data of reaction <1>. It has been verified that in the presence of the molecular sieve based catalyst according to the invention, beginning at 220 °C the maximum possible conversion is practically reached, thus an approach to the thermodynamic equilibrium higher than 95 %, whereas the conversion of methanol over alumina at 250°C is only about 60 %.

By using this catalyst according to the present invention it is not necessary to operate at temperatures higher than 250 °C, this is an improvement if compared to the alumina or alumina silicate based catalysts, for which temperatures exceeding 300 °C are necessary and has the further advantage that no hydrocarbon byproducts are formed at the lower temperatures.

## Example 4

86.2 g of 99.9 % methanol and 15.8 g recycled methanol containing 12 % water were fed at 270 °C and 8 bar over 50 g of catalyst according to example 2.

A conversion of methanol of 86.1 % is obtained, but the reaction products also contains 0.2 % wt of hydrocarbons having the composition as given in Table 3. No compounds having more than 5 carbon atoms are formed, thus there are no aromatic hydrocarbons in the product mixture. The methane content in the hydrocarbon byproduct is only of 2.28 % wt, which is equivalent, when taken as a ratio to the DME before the distillation to the 0.005 %, thus 50 ppm.

Table 3 Composition of the hydrocarbon fraction.

| Component | Weight % |
|---|---|
| Methane | 0.92 |
| Hydrogen | 2.28 |
| Ethane | 22.37 |
| Propane | 3.76 |
| Propylene | 62.25 |
| Butane | 8.24 |
| i-Pentane | 0.18 |

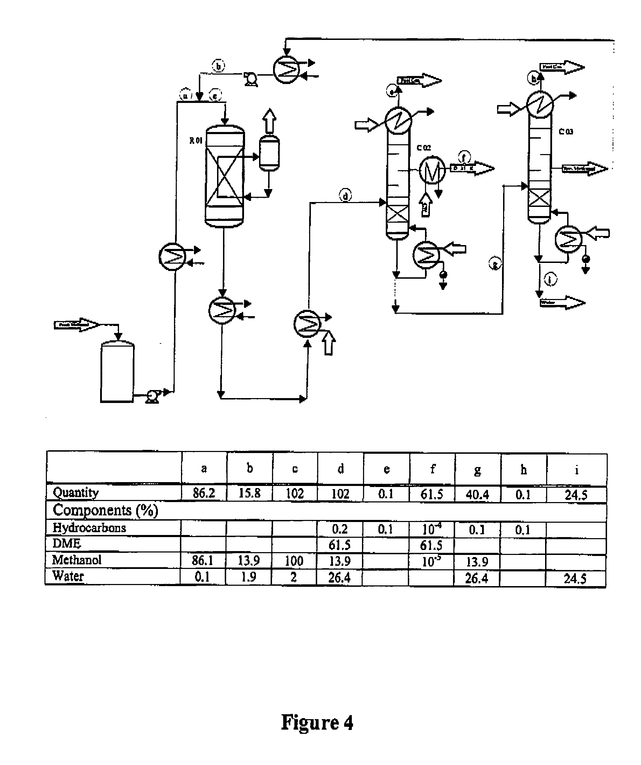

In particular, the process flow sheet is shown in figure 4 which also gives the material balance with reference to a 100 kg of 100% methanol feed to the reactor.

As can be seen from figure 4, the methanol feed, directed to the reactor **R 01** through the stream **a,** is mixed with recycled methanol (also containing water) coming from the stream **b** and the resulting stream enters the reactor **R01** for methanol conversion to DME. The reactor **R 01** operates at 8 bar and 260 °C. The reaction products exit from reactor **R 01** in the proportions as indicated by stream d and are then directly conveyed to the 10th tray of column **C 02,** that has a total of 23 theoretical stages (TS). From the 18th tray a side stream is drawn off consisting of 61.5 kg of anhydrous DME containing as impurities 100 ppm of hydrocarbons $C_3$-$C_4$ and 10 ppm of methanol. The product has thus a purity of 99.999 %. The overhead product of column **C02** consists of 0,1 parts of $C_1$-$C_2$ hydrocarbons and hydrogen, the bottom stream from column **C02** consists of 40.4 kg of methanol-water and $C_3$-$C_5$ hydrocarbons, with concentrations as given in stream **g.** Said mixture is fed to column **C03,** that operates at atmospheric pressure, and has a total of 18 TS. The feed to the column is on the 10th tray, from the 6th tray 15.8 kg are drawn off as a side stream (stream **b**). The stream **b** is a mixture of methanol and water with a concentration of 88 % wt methanol and contains no hydrocarbons. Said stream is recycled to reactor **R01** for the synthesis of DME. The overhead product of column **C03** consists of 0.1 kg of an organic fraction of $C_3$-$C_4$ hydrocarbons and the bottom stream is very pure water that may be used to generate the steam necessary for the columns operation. The process gives no wastewaters, and does not generate gaseous effluents to the atmosphere since the overhead streams of the two columns are recovered and used as liquefied gas fuels.

The chemical reaction from methanol to DME takes place without volume change, so the pressure does not change the proportions between the components that results from the process, but only the plant production, since the contact time of products with catalyst has to be maintained. The reactor pressure is chosen as a function of the cooling medium availability for the heat exchange with the column *C02* side stream, i.e. the DME product.

For the example the most economic solution has been chosen, i.e. the cooling with recycle water at 28 °C, therefore the product has to be at a pressure of 8 bar.

## Example 5

A molecular sieve for a catalyst according to the invention was prepared according to the procedure of example 1 except that the calcination was carried out in the presence of an inert gas (argon) at a temperature up to 500°C. It was surprisingly found that the calcination in inert gas is more efficient in removing completely the template at milder conditions.

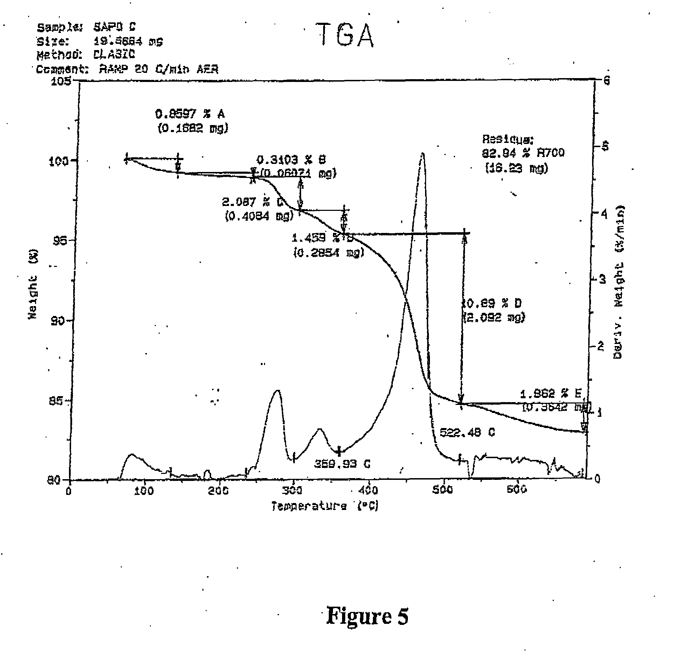

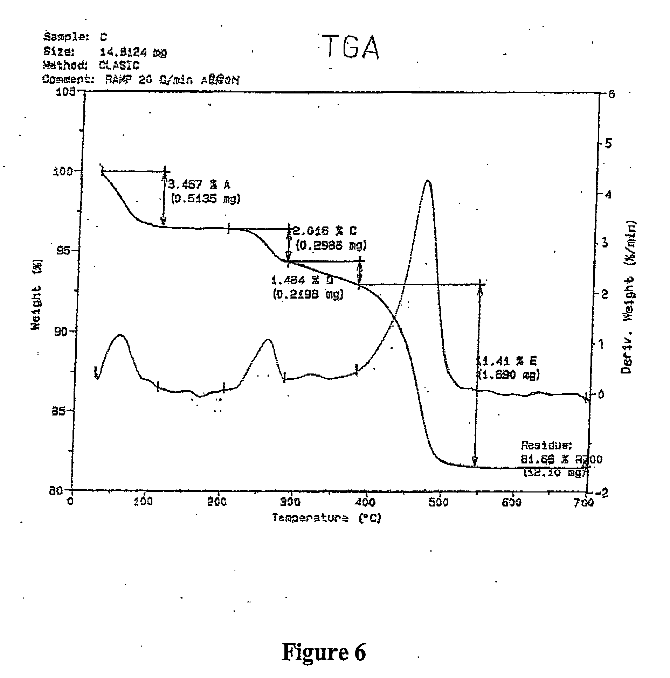

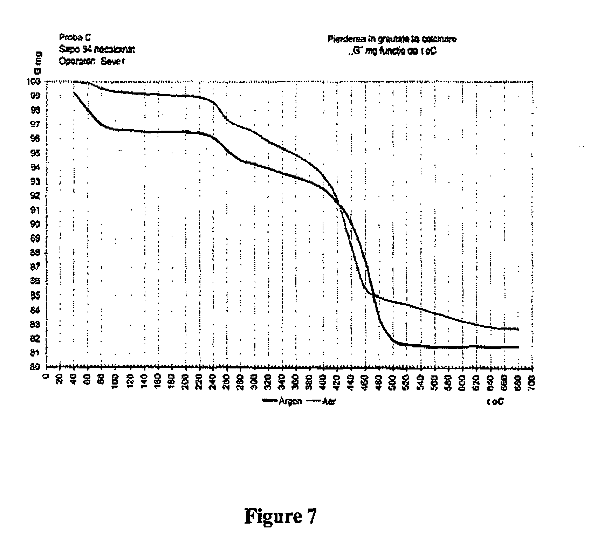

As can be seen in Figure 5, the thermal analysis of as synthesised molecular sieve sample obtained according to example 1 in air shows continuous weight loss after 550 °C till 700 °C. Thermogravimetric curves made in inert medium, Argon of the molecular sieve according to this example (Figure 6) exhibit total organic material removing at 550°C. The superposition of the weight loss curves in Figure 7 shows more clearly these differences.

## Example 6

The catalyst lifetime was studied at a methanol conversion around 70 % on a catalyst prepared according to example 1. A start reaction temperature of 220 °C was used in the reactor.

The duration test was made using 10 ml catalyst and atmospheric pressure. The termination criteria were set for the production of highly pure DME, thus selectivity level of 99.9%.

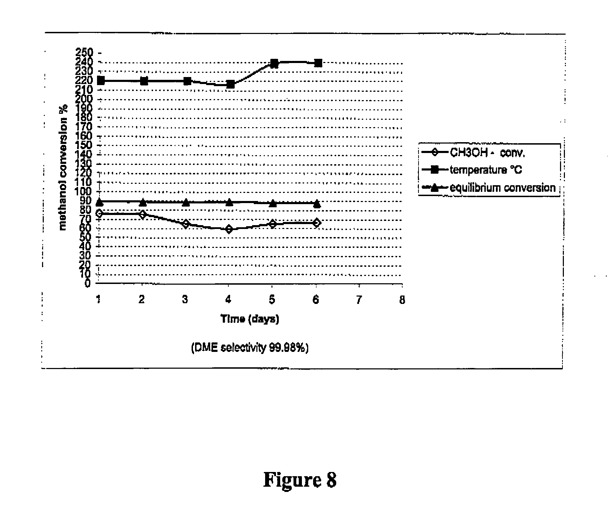

The conversion was followed and maintained during six days. Day 5 and day 6 the temperature was raised to 240 °C. The experimental data are presented in Figure 8. At this temperature the selectivity level was still above 99.9 %.

The examination of the catalyst after the reaction active cycle shows only the blocking of the active centers with oligomers.

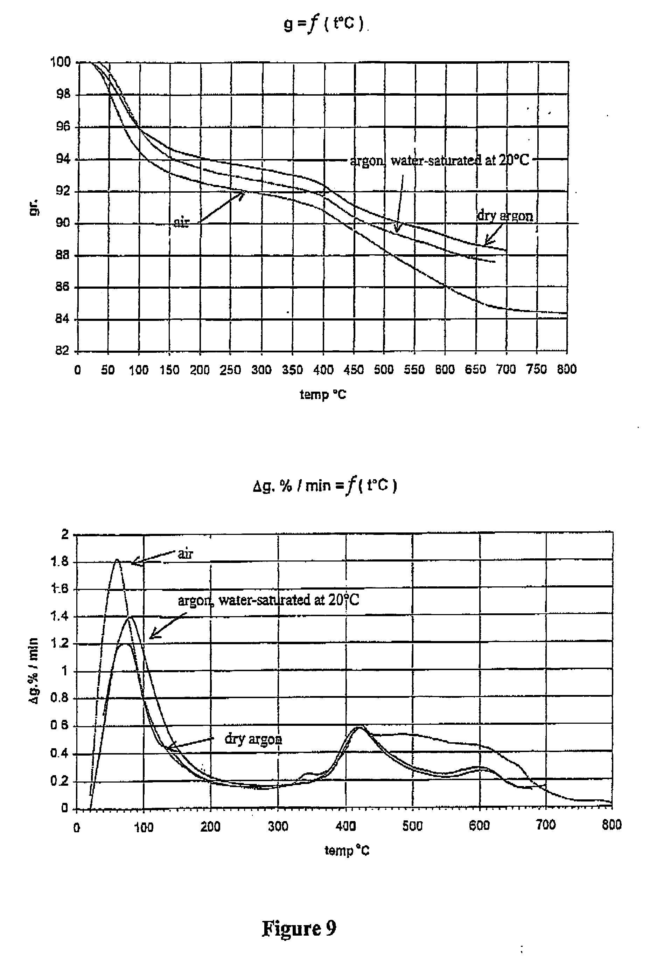

The low organic material deposited in the catalyst pores, as indicated by the thermal analysis (Figure 9) and lack of coke show that the catalyst regeneration can be made in mild conditions. The thermal analysis of the used catalyst sample in air shows a continuous weight loss after 550 °C. The thermogravimetric (TG) analysis in argon, and also in wet argon shows that the weight loss is almost finished at 550 °C. The differential thermogravimetric courves (figure 9) show a well defined peak between 400-540 °C for regeneration in Argon, an inert medium, or wet Argon and a broad one in the domain 400-700 °C for air. The TG measurements were made with a high temperature ramp of 20 °C/min, so in Argon the catalyst regeneration is finished in 23 minutes, a very short time.

## Example 7

The catalyst of Example 6 was regenerated according to the procedure given herebelow. The activity of the regenerated catalyst is presented in Table 4. As can be seen the activity of the catalyst is completely restored.

The regeneration was performed in the following way. After a catalyst active cycle in a reactor, the pressure in the reactor was reduced to regeneration regime till 1-2 bar and the catalyst having a temperature of 260-270°C was purged with nitrogen for one hour at nitrogen purge 100 N1 / $l_{catalyst}$ hour. The temperature was then raised to at 500 °C in 100 $h^{-1}$ nitrogen flow, during 4 hours. The nitrogen was then stepwise replaced with air or air/steam mixture at the same space velocity. The temperature was maintained at 500 °C for 4-8 hours in 200 $h^{-1}$ air or air/steam flow and then decreased in the same air or air/steam flow space velocity to the initial catalyst reaction temperature (175-220 °C) in about 4 hours. The catalyst was then purged again with two hours 100 $h^{-1}$ nitrogen purge at the initial catalyst reaction temperature. Finally, the reaction was restarted by stepwise replacing nitrogen with methanol in 2-4 hours till the normal methanol feed in the process. In this step the temperature does not exceed the initial catalyst reaction temperature.

**Table 4**: activity of the catalyst after regeneration

| Catalyst | Example 1 Batch 02-Cl-4 |
| --- | --- |
| Temperature °C | 220 |
| Pressure atm | 9 |
| Feed methanol ml/h | 68.7 |

(continued)

| Catalyst | Example 1 Batch 02-Cl-4 |
|---|---|
| Liquid reaction products ml/h | 16 |
| Composition wt % DME | 5.36 |
| methanol | 48.07 |
| water | 46.57 |
| Methanol conversion % | 77.5 |
| Gaseous product composition wt % C1-C2 hydrocarbons | 0.02 |
| propylene | 0.01 |
| DME | 99.96 |
| C4 + | 0.01 |
| DME selectivity % | 99.96 |

**Claims**

1. Process for the production of DME **characterized by** the step of dehydrating methanol in vapor phase at a temperature in the range from 160°C to 280°C in the presence of a catalyst comprising, as an active component, a silicoaluminophosphate (SAPO) molecular sieve having a minimum medium acidity of 0.5-1 mmol for gram of dry sieve.

2. Process according to claim 1, **characterized in that** said SAPO molecular sieve is mixed with a matrix in a ratio of 2-30% by weight, preferably 10-30% by weight, said matrix being chosen by the group comprising activated alumina, silica, aluminium silicate, silica-alumina and natural caoline.

3. Process according to claim 1 or claim 2, **characterized in that** said dehydration step is carried out at temperatures in the range from 160°C to 250°C while preferably 180°C to 220°C.

4. Process according to anyone of the preceding claims, **characterized in that** the methanol feed is a vapor flow of commercial methanol having a minimum methanol content of 99.8% or a vapor flow comprising methanol as a main component, preferably a mixture of methanol and steam.

5. Process according to any one of the preceding claims, **characterized in that** said SAPO molecular sieve is a SAPO-34 molecular sieve.

6. Process according to any one of the preceding claims, **characterized in that** said dehydration step is carried out in a reactor having a fixed bed or a fluidized bed of said catalyst.

7. Process according to claim 6, **characterized in that** the operating pressure of said reactor is in the range from atmospheric pressure to 20 bar, preferably 5 to 15 bar.

8. Process according to any of the claims 4 to 7, **characterized in that** said methanol flow has a space velocity, expressed as liters of liquid methanol supplied for liters of catalyst volume, in the range of 1 to 5, preferably 1.5 to 3.5.

9. Process according to any of the claims 4 to 8, **characterized in that** said methanol flow has a linear velocity in the range of 1 to 5 cm/sec, preferably from 1.5 to 3.5 cm/sec.

10. Catalyst for the conversion of methanol to DME **characterized in that** it comprises, as an active component, a silicoaluminophosphate (SAPO) molecular sieve having a medium acidity of 0.5-1 mmol for gram of dry sieve.

11. Catalyst according to claim 10, **characterized in that** said SAPO molecular sieve is mixed with a matrix.

12. Catalyst according to claim 11, **characterized in that** it comprises 70-98% by weight of said matrix and 2-30% by weight of said SAPO molecular sieve, preferably, 90-98% by weight of said matrix material and 2-10% by weight of said SAPO molecular sieve.

13. Catalyst according claim 11 or 12, **characterized in that** the material of said matrix is chosen by the group comprising activated alumina, silica, aluminium silicate, silica-alumina and natural caoline.

14. Catalyst according to claim 13, **characterized in that** said matrix material is activated aluminas having at least 50% by weight of pseudoboehmite and more than 100 $m^2$/g surface area.

15. Catalyst according to any one of the preceding claims 10 to 14, **characterized in that** it is in the form of pellet or extrudates having 1-6 mm diameter and 1-5 mm length.

16. Catalyst according to any one of the preceding claims 10 to 14, **characterized in that** it is in the form of microspheres having medium diameter form 0.05 to 0.08 mm.

17. SAPO molecular sieve **characterized by** having a medium acidity of 0.5-1 mmol for gram of dry sieve.

18. SAPO molecular sieve according to claim 17 **characterized in that** it is a SAPO-34 molecular sieve.

19. Process for preparing a catalyst according to any one of the claims 10 to 16 comprising the steps of:

- preparing a mixture comprising as reagents A1 source, silica sol, phosphoric acid and a templating agent,
- hydrothermal treating said mixture to a temperature of 190-200 to obtain a SAPO molecular sieve suspension,
- recovering said SAPO molecular sieve from the suspension and drying it,
- mixing said dried SAPO molecular sieve with a matrix to obtain a paste,
- treating said paste to obtain solid particles in the form of pellets, extrudates or microspheres,
- calcinating said solid particles at a temperature up to 500-600°C, so obtaining said catalyst.

20. Process according to claim 19, wherein said Al source is alumina or aluminium nitrate.

21. Process according to claim 19 or claim 20, wherein said templating agent is TEAOH.

22. Process according to claim 21, wherein in the preparation of said mixture TEAOH is first mixed with phosphoric acid aqueous solution to obtain tetraethylammonim phoshate (TEAP) in aqueous solution and then Al source and silica sol are added to the latter solution.

23. Process according to claim 22, wherein the molar ratios of said reagents in the final mixture expressed in terms of P, Al and Si oxides and using TEAOH as a templating agent are: $Al_2O_3$ : $SiO_2$ : TEAOH : $H_2O$ = 1 : (1.0-1.4): (0.2-0.4) : (1.0-1.15) : (110-130)

24. Process according to anyone of the claims 19 to 22, wherein all said reagents have a Na content below 0.01 %.

25. Process according to anyone of the claims 19 to 24, wherein said calcination is started at ambient temperature and the temperature is gradually increased in 4-10 hours up to 600°C and kept at 600°C for further 4-8 hours.

26. Process according to anyone of the claims 19 to 24, wherein said calcination is performed in an inert gas up to a temperature of 500°C.

27. Process according to claim 26, wherein said inert gas is argon or nitrogen.

28. Process for regenerating the catalytic activity of a catalyst according to any one of the claims 10 to 16 after each cycle of its use in a process according to any one of the claims 1-9, the regenerating process comprising the step of passing across the catalyst at least one flow of a regenerating agent chosen among nitrogen, argon, hydrogen, air, steam and mixtures thereof at a temperature of maximum 500°C for a period from 6 to 12 hours.

29. Process according to claim 28, comprising the steps of passing across said catalyst a flow of nitrogen and then a flow of air or air/steam.

Figure 1

**Figure 2**

**Figure 3**

| | a | b | c | d | e | f | g | h | i |
|---|---|---|---|---|---|---|---|---|---|
| Quantity | 86.2 | 15.8 | 102 | 102 | 0.1 | 61.5 | 40.4 | 0.1 | 24.5 |
| Components (%) | | | | | | | | | |
| Hydrocarbons | | | | 0.2 | 0.1 | $10^{-4}$ | 0.1 | 0.1 | |
| DME | | | | 61.5 | | 61.5 | | | |
| Methanol | 86.1 | 13.9 | 100 | 13.9 | | $10^{-5}$ | 13.9 | | |
| Water | 0.1 | 1.9 | 2 | 26.4 | | | 26.4 | | 24.5 |

# Figure 4

Figure 5

**Figure 6**

**Figure 7**

**Figure 8**

$$g = f\,(\,t°C\,)$$

$$Δg.\ \%\,/\,min = f(\,t°C\,)$$

**Figure 9**

**EP 1 932 592 A1**

**European Patent Office**

**EUROPEAN SEARCH REPORT**

Application Number

EP 06 02 5797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | POP G ET AL: "SAPO-34 CATALYST FOR DIMETHYLETHER PRODUCTION" STUDIES IN SURFACE SCIENCE AND CATALYSIS, ELSEVIER SCIENCE B.V., AMSTERDAM, NL, vol. 130A, 9 July 2000 (2000-07-09), pages 287-292, XP001023586 ISSN: 0167-2991 * the whole document * | 1-27 | INV. B01J29/85 B01J29/90 C01B37/08 C07C41/09 C07C43/06 |
| X | DATABASE CAPLUS [Online] CHEMICAL ABSTRACTS SERVICE, COLUMBUS, OHIO, US; POP, GRIGORE: "Process and zeolitic methanol etherification catalysts for the preparation of high-purity dimethyl ether for use as an aerosol propellant" XP002439049 retrieved from STN Database accession no. 1999:304313 * abstract * -& RO 107 249 B1 (ROM.) 30 October 1993 (1993-10-30) | 1-27 | |
| X | US 2003/232006 A1 (CAO GUANG [US] ET AL) 18 December 2003 (2003-12-18) * page 9, paragraph 110 - paragraph 112 * | 28,29 | TECHNICAL FIELDS SEARCHED (IPC) B01J C01B C07C |
| X | WO 01/66497 A (EXXONMOBIL CHEM PATENTS INC [US]) 13 September 2001 (2001-09-13) * page 8, line 30 - line 27 * | 28,29 | |
|  | -/-- | | |

The present search report has been drawn up for all claims

| Place of search | Date of completion of the search | Examiner |
|---|---|---|
| Munich | 25 June 2007 | Seufert, Gudrun |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

21

**European Patent Office**

**EUROPEAN SEARCH REPORT**

Application Number

EP 06 02 5797

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHAO S-Q ET AL: "THE ACIDITY AND CATALYTIC ACTIVITY OF SAPO-34 CATALYSTS" JOURNAL OF NATURAL GAS CHEMISTRY, CHENGDU INSTITU OF ORGANIC CHEMISTRY, ACADEMIA SINICA, vol. 2, no. 4, 1993, pages 315-320, XP001023604 * the whole document * | 1-29 | |
| A | WO 00/74846 A (EXXON CHEMICAL PATENTS INC [US]) 14 December 2000 (2000-12-14) * claims * | 1-29 | |

TECHNICAL FIELDS SEARCHED (IPC)

The present search report has been drawn up for all claims

| Place of search | Date of completion of the search | Examiner |
|---|---|---|
| Munich | 25 June 2007 | Seufert, Gudrun |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

## ANNEX TO THE EUROPEAN SEARCH REPORT
## ON EUROPEAN PATENT APPLICATION NO.

EP 06 02 5797

This annex lists the patent family members relating to the patent documents cited in the above-mentioned European search report.
The members are as contained in the European Patent Office EDP file on
The European Patent Office is in no way liable for these particulars which are merely given for the purpose of information.

25-06-2007

| Patent document cited in search report | | Publication date | Patent family member(s) | | Publication date |
|---|---|---|---|---|---|
| RO 107249 | B1 | 30-10-1993 | NONE | | |
| US 2003232006 | A1 | 18-12-2003 | AT | 312057 T | 15-12-2005 |
| | | | AU | 2003225144 A1 | 31-12-2003 |
| | | | DE | 60302664 T2 | 20-07-2006 |
| | | | EP | 1511689 A1 | 09-03-2005 |
| | | | ES | 2250888 T3 | 16-04-2006 |
| | | | WO | 03106343 A1 | 24-12-2003 |
| | | | US | 2003232718 A1 | 18-12-2003 |
| | | | ZA | 200408845 A | 26-10-2005 |
| | | | ZA | 200408846 A | 01-11-2005 |
| WO 0166497 | A | 13-09-2001 | AT | 301628 T | 15-08-2005 |
| | | | AU | 780552 B2 | 24-03-2005 |
| | | | AU | 5171101 A | 17-09-2001 |
| | | | CA | 2399213 A1 | 13-09-2001 |
| | | | CN | 1419527 A | 21-05-2003 |
| | | | DE | 60112556 D1 | 15-09-2005 |
| | | | DE | 60112556 T2 | 08-06-2006 |
| | | | EP | 1263700 A1 | 11-12-2002 |
| | | | ES | 2244610 T3 | 16-12-2005 |
| | | | MX | PA02008735 A | 12-03-2003 |
| | | | NO | 20024269 A | 15-10-2002 |
| | | | ZA | 200207145 A | 05-05-2003 |
| WO 0074846 | A | 14-12-2000 | AT | 264140 T | 15-04-2004 |
| | | | AU | 778004 B2 | 11-11-2004 |
| | | | AU | 5323200 A | 28-12-2000 |
| | | | BR | 0011426 A | 26-03-2002 |
| | | | CA | 2371091 A1 | 14-12-2000 |
| | | | CN | 1414882 A | 30-04-2003 |
| | | | DE | 60009902 D1 | 19-05-2004 |
| | | | DE | 60009902 T2 | 14-04-2005 |
| | | | EP | 1189696 A2 | 27-03-2002 |
| | | | ES | 2215673 T3 | 16-10-2004 |
| | | | JP | 2003501233 T | 14-01-2003 |
| | | | MX | PA01012625 A | 21-06-2002 |
| | | | NO | 20015973 A | 07-02-2002 |
| | | | TW | 572783 B | 21-01-2004 |
| | | | ZA | 200110057 A | 09-07-2003 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 4590320 A
- US 6936566 B
- US 6514899 B

### Non-patent literature cited in the description

- **G.H. HUTCHINGS ; R. HUNTER.** *Catal. Today,* 1990, vol. 6, 279
- **H. SCHULZ ; W. BOEHRIGEREN ; S. ZHAO.** *Proceedings of the 9th Int. Molecular sieve Conf,* 1993, vol. 2, 567
- *Applied Catalysis A,* 2004, vol. 276, 251-255
- *Fuel Processing technology,* 2003, vol. 83, 203-218
- *Microporous and Mesoporous Materials,* 1999, vol. 29, 3-48
- *Catalysis communications,* 2005, vol. 6, 147-152